# EUROPEAN PATENT APPLICATION

(11) **EP 0 582 400 A1**
(43) Date of publication of application: **09.02.1994**
(21) Application number: 93305712.7
(22) Date of filing: 20.07.1993
(51) Int. Cl.: A61J 15/00, A61B 5/042

(54) **Medico-surgical assemblies**

(30) Priority: 05.08.1992 GB 9216615
(71) Applicant: Smiths Industries Public Limited Company, London, NW11 8DS (GB)
(72) Inventor: Carter, Roland Henry Clyne, Hythe, Kent CT21 6DW (GB)
(74) Representative: Flint, Jonathan McNeill

(57) **Abstract**

A naso-gastric feeding tube 10 has an ECG monitoring electrode 15 connected to a wire 16 that extends along the bore 14 of the tube. At its machine end, the tube 10 and wire 16 are bent into a side arm 26 of a Y-piece coupling 20 moulded about the tube. An opening 18 in the wall of the tube 10 enables feeding fluid to be supplied to the tube via the other arm 23 of the coupling.

## Description

This invention relates to medico-surgical assemblies of the kind including a tube having a fluid passageway extending along its length and an elongate signal-carrying element extending along the length. of the tube.

The invention is more particularly concerned with catheters or other medico-surgical tube assemblies incorporating an elongate signal-carrying element, such as an electrically-conductive element for supplying current to or from an electrode or sensor in the assembly.

Catheters are known in which a conductive wire or other element extends along the length of the catheter within its bore or within its wall. At the tip of the catheter, the wire is connected to an electrode on the external surface of the catheter so that electrical impulses in body tissue contacted by the electrode are supplied along the wire, such as for use in cardiac monitoring. Examples of catheters having an electrode are shown in WO 9217150 and GB 9312292. Alternatively, the wire could be connected to a sensor, such as a temperature sensor.

The bore of the catheter is used as a fluid passage for supply of fluid along the catheter to or from a body site, or to an inflatable balloom. At the proximal, machine end of the assembly it is necesary to make electrical connection to the conductive element and to make fluid connection to the bore along the catheter. This can present problems, especially where the catheter has a small diameter bore and where it is necessary to keep the cost of the catheter assembly as low as possible so that it can be a single-use, disposable product.

Similar problems exist with assemblies in which the signal-carrying element is in the form of a fibre-optic cable.

It is an object of the present invention to provide an improved medico-surgical assembly and a method of making such an assembly.

According to the present invention there is provided a medico-surgical assembly of the above-specified kind, characterised in that the assembly includes a Y-piece mounted at the machine end of the tube, that the tube and the signal-carrying element extending into a first arm of the Y-piece, and that the tube has an opening formed in its wall located in alignment with the second arm of the Y-piece such that fluid connection with the assembly can be made via the second arm of the Y-piece and the opening and such that signal connection with the assembly can be made via the first arm.

The signal-carrying element may extend within the fluid passageway of the tube. The first arm of the Y-piece may be a side arm that extends at an angle away from the axis of the Y-piece, the second arm being aligned with the axis of the Y-piece. The signal-carrying element may extend out of the tube and be folded back to lie between the outside of the tube and the inside of the first arm. The signal-carrying element is preferably an electrical wire. The assembly may include an electrode on an outer surface of the tube, the signal-carrying element extending to the electrode. The Y-piece is preferably moulded about the machine end of the tube.

A paediatric naso-gastric feeding tube assembly with an ECG electrode, in accordance with the present invention, will now be described, by way of example with reference to the accompanying drawings, in which:
- Figure 1: is a partly sectional side elevation of the assembly;
- Figure 2: is an enlarged sectional side elevation of a part of the assembly shown in Figure 1; and
- Figure 3: is a sectional side elevation of an alternative assembly.

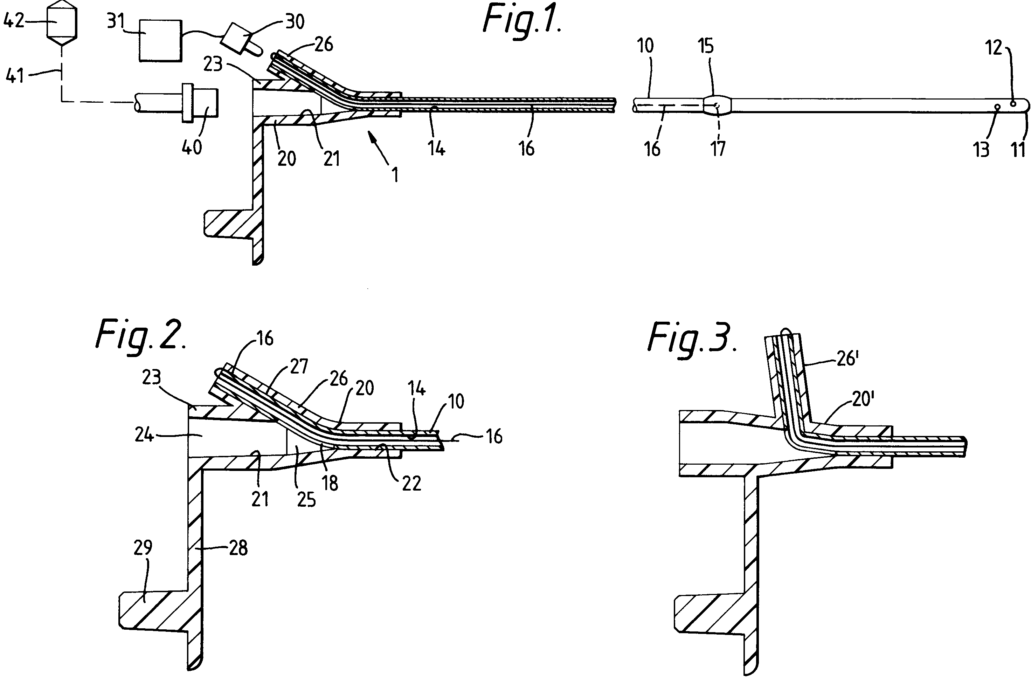

With reference to Figures 1 and 2, the catheter assembly 1 is used in paediatric naso-gastric feeding and ECG monitoring. The assembly 1 includes a PVC tube 10 with a length of 770mm, an external diameter of 1.98mm and an internal diameter of 1.37mm. At its patient, distal end 11, the tube is closed and provided with a rounded tip. Two side apertures 12 and 13 are provided through the wall of the tube 10 into its bore 14 at distances respectively of 2.5mm and 5mm from the end 11.

The tube 10 also has an electrode 15 at a location about 50mm from the patient end 11. The electrode 15 extends as an annular ring around the outside of the tube and has a convex external profile. The electrode 15 is made from a conductive plastics such as PVC loaded with 30% carbon and is moulded in position on the tube. One end of a stainless steel wire 16 of diameter 0.15mm extends through an aperture 17 in the wall of the tube 10 underlying the electrode 15 and is electrically connected to the electrode. The wire 16 extends rearwardly from the electrode 15 along the bore 14 of the tube 10 to its machine or proximal end where the tube is joined to a Y-piece 20 forming a part of the assembly 1.

The Y-piece 20 is an integral, one-piece PVC moulding with an axial bore 21 extending along its length. The forward, distal end 22 of the bore 21 has a diameter equal to the external diameter of the tube 10. At its rear, proximal end, the bore 21 extends into the main, fluid arm 23 of the Y-piece and has an enlarged section 24 with a taper of about 3 degrees and an opening of diameter 3.6mm. The forward and rear sections 22 and 24 of the bore 21 are connected via an intermediate tapering section 25. A side arm 26 extends from the Y-piece 20 at an angle of about 30 degrees from the axis. The side arm 26 has a bore 27 through it which opens into the tapering section 25 about midway along the length of the Y-piece 20. The diameter of the bore 27 is the same as the external diameter of the tube 10, the length of the side arm along its longer edge being 10.8mm. A flexible web 28 extends from the side of the Y-piece remote from the side arm 26. The web 28 supports a stopper 29 the dimensions of which are such that it can be inserted into the proximal end of the enlarged section 24 of the bore 21, as a push fit.

The proximal end of the tube 10 extends into the bore 21 at the distal end of the Y-piece 20 and bends upwardly into the bore 27 through the side arm 26, terminating level with the end of the side arm. The wire 16 extends along the tube 10 within the side arm 26 and is folded back a short distance around the outside of the tube 10 so that is is trapped between the outside of the tube and the inside of the side arm. An elliptical opening 18 is formed through the wall of the tube 10 on the outside of the bend in the tube. The shape and size of the opening 18 are chosen such that its perimeter follows the point of contact of the external surface of the tube with the inside of the Y-piece 20. The Y-piece is preferably moulded in place about the tube 10 but may alternatively be secured to the tube by means of a solvent, adhesive or by heat treatment.

In use, a male electrical plug 30 is inserted in the proximal end of the tube 10 so that it makes contact with the wire 1. The plug 30 connects the assembly with an ECG monitor 31 and also serves to provide a fluid-tight seal with the proximal end of the bore 14 through the tube 10. Alternatively, the bore 14 through the tube 10 can be sealed at a location between its proximal, machine end and the opening 18 by means of an insert bonded into the tube.

A fluid coupling 40 is inserted into the main axial arm of the Y-piece 20, the fluid coupling being connected via tubing 41 to a bag 42 of feeding liquid. Liquid in the bag 42 flows into the Y-piece 20 and into the tube 10 via the opening 18. The liquid flows along the tube 10 and out through the two distal apertures 12 and 13.

The simple construction of the assembly enables it to be made at low cost and provides a reliable electrical connection between the ECG monitor 31 and the electrode 15.

Various alternative constructions are possible.

For example, instead of a metal wire, the assembly could have an electrically-conductive strip extruded within the wall of the tube. The strip could be of PVC loaded with 30% carbon. If the strip were exposed on the outside of the tube, a separate electrode might not be needed.

The side arm 26' of the Y-piece 20' could be inclined at a greater angle to the axis as shown in Figure 3 where the arm extends at an angle of about 80 degrees. This has the advantage of spacing the end of the side arm 26' further from the machine end of the Y-piece so that access to the side arm and the main arm is improved.

The invention need not be used for ECG monitoring but it could instead, for example, be used for supplying current signals to the body such as, for example, for pacemaking or muscle stimulation applications. Alternatively, the assembly could include a sensor, such as, for example, a temperature sensor connected to an external monitor by means of the wire or other signal-carrying element.

The invention could be used for assemblies having an optical fibre extending along a tube or catheter. In this case, an alternative form of connection at the side arm would be needed.

## Claims

1. A medico-surgical assembly including a tube (10) having a fluid passageway (14) extending along its length and an elongate signal-carrying element (16) extending along the length of the tube, characterised in that the assembly includes a Y-piece (20) mounted at the machine end of the tube (10), that the tube (10) and the signal-carrying element (16) extend into a first arm (26) of the Y-piece (20), and that the tube (10) has an opening (18) formed in its wall located in alignment that a second arm (23) of the Y-piece (20) such that fluid connection with the assembly can be made via the second arm of the Y-piece and the opening (18) and such that signal connection with the assembly can be made via the first arm (26).

2. An assembly according to Claim 1, characterised in that the signal-carrying element (16) extends within the fluid passageway (14) of the tube (10).

3. An assembly according to Claim 1 or 2, characterised in that the first arm (26) of the Y-piece (20) is a side arm that extends at an angle away from the axis of the Y-piece.

4. An assembly according to any one of the preceding claims, characterised in that the second arm (23) of the Y-piece (20) is aligned with the axis of the Y-piece.

5. An assembly according to any one of the preceding claims, characterised in that the signal-carrying element (16) extends out of the tube (10) and is folded back to lie between the outside of the tube (10) and the inside of the first arm (26).

6. An assembly according to any one of the preceding claims, characterised in that the signal-carrying element (16) is an electrical wire.

7. An assembly according to any one of the preceding claims, characterised in that the assembly includes an electrode (15) on an outer surface of the tube (10), and that the signal-carrying element (16) extends to the electrode.

8. An assembly according to any one of the preceding claims, characterised in that the Y-piece (20) is moulded about the machine end of the tube (10).
